# EUROPEAN PATENT SPECIFICATION

(11) **EP 2 564 848 B1**
(45) Date of publication and mention of the grant of the patent: **02.09.2015**
(21) Application number: 11775069.5
(22) Date of filing: 27.04.2011
(51) Int. Cl.: A61K 31/445, A61K 9/70, A61K 47/28, A61P 25/28

(54) **TRANSDERMALLY ABSORBABLE DONEPEZIL-CONTAINING PREPARATION**
TRANSDERMAL ABSORBIERBARE DONEPEZILHALTIGE ZUBEREITUNG
PRÉPARATION CONTENANT DU DONÉPÉZIL ABSORBABLE PAR VOIE TRANSDERMIQUE

(30) Priority: 28.04.2010 JP 2010104120
(43) Date of publication of application: 06.03.2013
(73) Proprietor: Hisamitsu Pharmaceutical Co., Inc., Tosu-shi, Saga 841-0017 (JP)
(72) Inventor: AIDA Kazunosuke, Tsukuba-shi Ibaraki 305-0856 (JP); MICHINAKA Yasunari, Tsukuba-shi Ibaraki 305-0856 (JP); ATARASHI Kenji, Tsukuba-shi Ibaraki 305-0856 (JP); TAKADA Yasunori, Tsukuba-shi Ibaraki 305-0856 (JP)
(74) Representative: Grünecker Patent- und Rechtsanwälte PartG mbB
(86) International application number: PCT/JP2011/060298
(87) International publication number: WO 2011/136288

(56) References cited:
- EP-A1- 0 958 823
- EP-A1- 1 437 130
- EP-A1- 2 514 416
- EP-A1- 2 564 873
- WO-A1-03/032960
- WO-A1-2009/075258
- WO-A1-2009/075258
- WO-A2-2008/021113
- JP-A- 11 315 016
- US-B1- 6 512 010
- MCCHESNEY J A: "Preventing the contact dermatitis caused by a transdermal clonidine patch.", THE WESTERN JOURNAL OF MEDICINE JUN 1991, vol. 154, no. 6, June 1991 (1991-06), page 736, XP002713388, ISSN: 0093-0415
- KAWAHARA KOJI ET AL: "Skin irritation in transdermal drug delivery systems: a strategy for its reduction.", PHARMACEUTICAL RESEARCH FEB 2007, vol. 24, no. 2, February 2007 (2007-02), pages 399-408, XP002713389, ISSN: 0724-8741
- KOKICHI MASUBUCHI SAIKIN NO HIFU GAIYOZAI 1991, NANZANDO, pages 195 - 198

## Description

### Technical Field

The present invention relates to a transdermally absorbable donepezil-containing preparation with reduced skin irritation, a manufacturing method thereof, and a method for reducing skin irritation of a transdermally absorbable donepezil-containing preparation.

### Background Art

Donepezil is an acetylcholinesterase inhibitor, which is considered to be effective in drug treatment for Alzheimer's dementia. Currently available donepezil preparations are only orally administered agents such as orally-disintegrating tablets and fine granules, containing donepezil hydrochloride. However, in the case of an oral preparation, metabolism/decomposition in liver cannot be avoided, and side effects in digestive system and transient and rapid increase in blood level may be observed.

Furthermore, the majority of patients with Alzheimer's dementia for whom therapeutic effects of donepezil are expected, are elderly. Therefore, constant bioavailability is difficult to obtain by oral administration because of decreased digestive system function, and furthermore, there may also be problems of non-compliance. In addition, the majority of patients with Down's syndrome and attention-deficit hyperactivity disorder, as well as Alzheimer's dementia, for which therapeutic effects of donepezil are expected, are children. For this reason, the side effects of donepezil may become severe, and the problems of non-compliance may also arise like in the case of the elderly.

In order to reduce the above-mentioned problems and side effects by oral preparations, some transdermally absorbable donepezil-containing preparation have been recently proposed as alternatives to the oral preparations. However, since skin does not generally allow drugs to penetrate, it is difficult to allow a therapeutically effective amount of drug absorbed from the skin into the body, and thus there are also problems of sufficient skin permeability of drug for the transdermally absorbable donepezil-containing preparation. Therefore, in Patent Literature 1, there are proposed transdermally-applied preparations containing, in addition to donepezil, a transdermal absorption enhancer selected from higher alcohols, lactic acid esters of higher alcohols, esters of higher fatty acids and lower alcohols, and esters of fatty acids having 6-18 carbon atoms and propylene glycol. In addition, in Patent Literature 2 it is described that a transdermal preparation containing donepezil comprising Type-B crystal donepezil and/or salt thereof in an amount of 9-50% by mass relative to total weight of adhesive agent has excellent skin permeability.

Although it has been well known that steroids with steroidal backbone generally have anti-inflammatory effects, for example, Patent Literature 3 describes that cholesterol have no anti-inflammatory activity. Furthermore, it is described in Patent Literature 4 that cholesterol are effective in suppressing skin irritation of a tape-type transdermal preparation (plaster) containing bisphosphonate. Percutaneous absorption preparations containing donezepil hydrochloride are also known from Patent Literatur 5 which mentions a high medicament skin permeation rate for the described preparation. Patent Literature 6 also describes a percutanous absorption preparation comprising an anti-dementia drug, which is preferably donezepil, wherein the preparation has a lower skin irritation.
In Patent Literature 7, a pharmaceutical preparation which contains azelastine hydrochloride as the drug is described. In order to reduce skin irritation, only additives known to have low irritation are used in the preparation.
Patent Literature 8 discloses as a preferred embodiment the transdermal administration of fluoxetine at reduced skin irritation levels wherein fluoxetine is coadministered with a corticosteroid.
Furthermore, in Patent Literature 9 a method for preventing contact dermatitis caused by a transdermal clonidine patch is disclosed which includes a pretreatment of the skin application site with an aerosolized spray medication containing beclomethasone dipropionate.

### Citation List

### Patent Literature

Patent Literature 1: U.S. Pat. No. 6,815,454B
Patent Literature 2: Japanese Patent Laid-Open No. 2007-302582
Patent Literature 3: Japanese Patent Laid-Open No. 1992-501415
Patent Literature 4: WO 2009/075258A
Patent Literature 5: EP 1437130A
Patent Literature 6: EP 2514416A
Patent Literature 7: EP 0958823A
Patent Literature 8: U.S. Pat. No. 6,512,010B
Patent Literture 9: J. McChesney, The Western Journal of Medicine 1991, 154(6), p. 736.

### Summary of Invention

### Technical Problem

However, the inventors have found that conventional transdermally absorbable donepezil-containing preparations might cause skin irritation such as pruritus, flushing, rash, pain, eczema and dermatitis in the topical skin to which the preparation was applied. Thus, to reduce skin irritation, the inventors decreased the content of donepezil in the transdermal preparation, and found that the skin irritation can be reduced, but donepezil skin permeability rate (absorbed amount within a given time) necessary for treatment cannot be ensured.

Thus, the object of an embodiment of the present invention is to provide a transdermal preparation containing one or more drugs selected from donepezil and pharmaceutically acceptable salts thereof, which has a low skin irritation as well as a sufficient skin permeability rate of donepezil.

### Solution to Problem

As a result of extensive and intensive studies to solve the above-mentioned problems, the inventors have found that a transdermal preparation containing donepezil and a skin irritation-reducing agent has a low skin irritation as well as a sufficient skin permeability rate of donepezil, thereby having completed the present invention.

An embodiment of the present invention is to provide a transdermal preparation which comprises one or more drugs selected from donepezil and pharmaceutically acceptable salts thereof, and a skin irritation-reducing agent. According to the transdermal preparation, it is possible to provide a transdermal preparation comprising one or more drugs selected from donepezil and pharmaceutically acceptable salts thereof, which has a drug skin permeability rate necessary for treatment, as well as a reduced skin irritation induced by the drug, when applied to the skin.

The skin irritation-reducing agent may be one or more cholesterol compounds selected from cholesterol, cholesterol derivatives and cholesterol analogs. The cholesterol derivative is an acylcholesterol which is an ester compound in which a fatty acid binds to the part of the hydroxy group, and the cholesterol analog is selected from sitosterol, stigmasterol, fucosterol, spinasterol, campesterol, brassicasterol, and ergosterol. Thereby higher skin permeability rate of donepezil can be achieved and skin irritation can be further reduced.

Content of the skin irritation-reducing agent may be 0.5-5% by mass relative to total amount of the transdermal preparation.

### Advantageous Effects

According to the present invention, it is possible to provide a transdermal preparation comprises one or more drugs selected from donepezil and pharmaceutically acceptable salts thereof, which has low skin irritation as well as a sufficient skin permeability rate of donepezil. The present invention can also provide a manufacturing method of a transdermally absorbable donepezil-containing preparation with reduced skin irritation, and a method for reducing skin irritation of a transdermally absorbable donepezil-containing preparation.

### Brief Description of Drawings

[Fig. 1] Fig. 1 illustrates a graph showing results of Experiment 1.
[Fig. 2] Fig. 2 illustrates a graph showing results of Experiment 3.

### Description of Embodiments

An embodiment of the present invention provides a transdermal preparation which comprises, at least, one or more drugs selected from donepezil and pharmaceutically acceptable salts thereof, and a skin irritation-reducing agent.

Donepezil (IUPAC name: 2-[(1-benzyl-4-piperidinyl)methyl] -5,6-dimethoxyindane-1-on) is considered to increase the amount of brain acetylcholine and activate the brain cholinergic nervous system, by reversibly inhibiting acetylcholinesterase which is an acetylcholine-degrading enzyme. Donepezil is a drug applied for inhibiting progress of Alzheimer's dementia. Drugs to be used may be free donepezil, or a pharmaceutically acceptable donepezil salt, or two or more combinations of them. The pharmaceutically acceptable salts are not particularly limited and may be inorganic salts or organic salts. The inorganic salts may include hydrochloride, bromate and silicate, and particularly hydrochloride is preferable. The organic salts may include acetate, citrate, fumarate and maleate, and particularly acetate is preferable.

In the transdermal preparation, the content of the one or more drugs selected from donepezil and pharmaceutically acceptable salts thereof may be a therapeutically effective amount, and although the content varies on the type of the transdermal preparation, generally the content may be 4-50% by mass, 5-30% by mass, furthermore 6-15% by mass relative to the total amount of the transdermal preparation. By these contents skin irritation caused by donepezil in the transdermal preparation can be reduced, and at the same time sufficient skin permeability rate of donepezil can be achieved. It should be noted that "total amount of the transdermal preparation" herein means the total mass of the drug-containing part. In the case of the dosage forms such as ointments, creams, gels, gelled creams, liniments and lotions, the total amount means the total mass, in the case of the dosage forms such as cataplasms, plasters and reservoir type patches, it means the mass of the part excluding the backing, and in the case of dosage forms such as sprays and aerosols, it means the mass of the part excluding the container part.

Immune reactions of epidermal cells are variously studied as one of the mechanisms causing skin irritation by drugs. Epidermal cells play a central role in cutaneous immunity by releasing, to the outside of the cells, many proinflammatory substances such as cytokine, chemokine, inflammatory mediator and cell growth factor, and by expressing cytokine receptor, adhesion factor and MHC class II on the cells ("Handbook of Cutaneous Immunity" published by CHUGAI IGAKUSHA).

For example, the proinflammatory substances released by epidermal cells include interleukin(IL)-1α, IL-10, IL-12, IL-18, TNF-α, GM-CSF, IL-6, IL-7, IL-15, TGF-α, amphiregulin, HB-EGF, bFGF, VEGF, PDGF, SCF, IFN-β, IFN-γ, TGF-β, MIP-3α, IP-9, IP-10, Mig, IL-8, GROa, RANTES, MCP-1, TARC, prostaglandin, leukotriene, substance P, reactive oxygen species and nitrogen oxide, and they cover a considerably broad range and intricately interact with each other to modulate immune reaction.

Thus, "reduction in skin irritation (reduced skin irritation)" herein means decreased production of a so-called skin irritation mediator induced by the drug, such as prostaglandin E2 (PGE2), IL-1α, IL-6 and IL-8, in an *in vitro* test using epidermal cells, and/or reduction in skin irritation induced by the drug in vivo, such as skin erythema/crust and swelling formation. The skin irritation may be evaluated by, for example, a skin primary irritation index (PII) as mentioned below.

As a skin irritation-reducing agent, there can be used, for example, aluminum hydroxide, titanium oxide, sucrose fatty acid ester, edetate, polyglyceryl fatty acid ester, hydroquinone glycoside, pantethine, tranexamic acid, lecithin, and steroidal and non-steroidal anti-inflammatory agents. The inventors have found that sterol compounds such as cholesterol, cholesterol derivatives and cholesterol analogs are particularly preferable as the skin irritation-reducing agent for donepezil.

Cholesterol is a (3β)-cholest-5-en-3-olcholest-5-en-3β-ol and is known as essential component in a cell membrane of a higher animal. The cholesterol derivative means a natural or synthetic cholesterol derivative and is exemplified by an acylcholesterol which is an ester compound in which a fatty acid binds to the part of the hydroxy group. In addition, the cholesterol analog means a natural or synthetic cholesterol analog and is exemplified by plant cell-derived phytosterols such as sitosterol, stigmasterol, fucosterol, spinasterol, campesterol and brassicasterol, and a fungous-derived ergosterol, etc.

All of the cholesterol, cholesterol derivatives and cholesterol analogs are classified into steroids, and, among them, belong to a subgroup referred to as sterol (steroid alcohol). The transdermal preparation may comprise any one of or a combination of two or more of these sterol compounds. Since these sterol compounds have effects of reducing the skin irritation induced by donepezil, the skin irritation induced by donepezil can be reduced by adding these compounds.

The content of the skin irritation-reducing agent varies on the type of the transdermal preparation, and may be usually 0.1-30% by mass, 0.3-10% by mass, 0.5-5% by mass, and furthermore 0.5-3% by mass relative to the total amount of the transdermal preparation. In the case of 0.1% by mass or less, sufficient effects of reducing the skin irritation may not be obtained, and in the case of 30% by mass or more, sufficient adhesiveness of the transdermal preparation may not be obtained.

It is preferable that the transdermal preparation further comprises a transdermal absorption enhancer. Transdermal absorption enhancers that can be used may be any of compounds in which the effects of promoting transdermal absorption in skins have been conventionally acknowledged. Examples of such promoting agents may be fatty acids having 6-20 carbon atoms, fatty alcohols, fatty acid esters, amides, ethers, aromatic organic acids, aromatic alcohols, aromatic organic acid esters and ethers (these compounds may be saturated or unsaturated, and may be circular, linear or branched), lactic acid esters, acetic acid esters, monoterpene compounds, sesquiterpene compounds, Azone, Azone derivatives, pirotiodecane, glycerine fatty acid esters, propylene glycol fatty acid esters, sorbitan fatty acid esters (Span series), polysorbate-based compounds (Tween series), polyethylene glycol fatty acid esters, polyoxyethlene hydrogenated castor oil-based compounds (HCO series), polyoxyethylene alkyl ethers, sucrose fatty acid esters and vegetable oils, etc.

Specifically, the examples can include caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetylalcohol, methyl laurate, hexyl laurate, lauric acid diethanol amide, isopropyl myristate, myristyl myristate, octyldodecyl myristate, cetyl palmitate, salicylic acid, methyl salicylate, salicylic acid ethylene glycol, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerin monocaprylate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene laurylether, HCO-60, pirotiodecane and olive oil. Above all, oleyl alcohol, lauryl alcohol, isostearyl alcohol, lauric acid diethanol amide, glycerin monocaprylate, glycerin monocaprate, glycerin monooleate, sorbitan monolaurate, polyethylene glycol monolaurate, polyoxyethylene laurylether and pyrrothiodecane are particularly preferable. Among them, a fatty acid having 6-20 carbon atoms is preferable, and oleic acid is particularly preferable. Two or more kinds of such transdermal absorption enhancers may be mixed for use.

By adding transdermal absorption enhancers it is possible to achieve sufficient skin permeability rate of donepezil. When oleic acid is added as the transdermal absorption enhancer, the content of oleic acid usually may be 0.1-20% by mass, 0.5-15% by mass, 5-10% by mass, and furthermore 6-8% by mass relative to the total amount of the transdermal preparation.

In the transdermal preparation, a mass ratio of the drugs to the sterol compounds may be 20:1-1:10, 10:1-1:5, and furthermore 5:1-1:1. These mass ratios make it possible to reduce skin irritation without affecting the skin permeability rate of donepezil. In addition, when oleic acid is added as a transdermal absorption enhancer, a mass ratio of the drugs to oleic acid may be 1:10-5:1, 1:5-3:1, and furthermore 1:2-2:1. These mass ratios make it possible to achieve sufficient skin permeability rate of donepezil while suppressing skin irritation.

Although the dosage form of the transdermal preparation is not particularly limited, the form may be ointment, cream, gel, gelled cream, liniment and lotion, spray, aerosol, cataplasm, plaster, reservoir type patch, etc. Above all, in view of adherability and absorbability, plaster and ointment, which are dosage forms substantially free from moisture, are preferable. However, it is acceptable that the drug-containing composition has only 1% by mass or less of water (moisture) derived from its raw materials or manufacturing environment.

As an embodiment of the transdermal preparation, a plaster will be explained below. Plaster comprises a backing, and a drug layer laminated on at least one side of the backing. The drug layer comprises one or more drugs selected from donepezil and its pharmaceutically acceptable salts, a skin irritation-reducing agent, and an adhesive base. The skin irritation-reducing agent is preferably one or more sterol compounds selected from cholesterol, cholesterol derivatives and cholesterol analogs. And the drug layer may further contain a transdermal absorption enhancer like oleic acid.

It is preferable that the drug layer of the plaster is substantially free from moisture. "Substantially free from moisture" means that the drug layer is composed of non-aqueous materials. However, it is acceptable that the drug layer contains only 1% by mass or less of water (moisture) derived from its raw materials or manufacturing environment.

The adhesive base is exemplified by an acrylic adhesive base, a rubber adhesive base, and a silicone adhesive base, etc. As the acrylic adhesive base, a single polymer or a copolymer of an alkyl (meth)acrylate ester having an alkyl group of 4-18 carbon atoms, or a copolymer of the above-mentioned alkyl (meth)acrylate ester and other functional monomer. It should be noted that the (meth)acryl means acryl or methacryl.

Specifically, as the adhesive base, there can be used an adhesive such as an acrylic acid/octyl acrylate ester copolymer, a 2-ethylhexyl acrylate/vinylpyrrolidone copolymer solution, an acrylic acid ester/vinyl acetate copolymer, a 2-ethylhexyl acrylate/2-ethylhexyl methacrylate/dodecyl methacrylate copolymer, a methyl acrylate/2-ethylhexyl acrylate copolymer resin emulsion, and an acrylic polymer contained in acrylic resin alkanolamine solution, which are listed in Dictionary of Pharmaceutical Excipients, 2000 (edited by International Pharmaceutical Excipients Council Japan) as adhesives, DURO-TAK acrylic adhesive series (Henkel), Eudragit series (Evonik Industries) and the like.

The acrylic adhesive base is not particularly limited as long as it containing a copolymer comprising at least one of (meth)acrylic acid derivative represented by 2-ethylhexyl acrylate, methyl acrylate, butyl acrylate, hydroxyethyl acrylate, 2-ethylhexyl methacrylate and the like, and above all, a copolymer comprising 50% or more of 2-ethylhexyl acrylate is preferable.

The rubber adhesive base includes styrene-isoprene-styrene block copolymer (SIS), isoprene rubber, polyisobutylene (PIB), styrene-butadiene-styrene block copolymer (SBS), styrene-butadiene rubber (SBR), polysiloxane and the like, above all, SIS, PIB and polysiloxanes are preferable, and SIS and PIB are particularly preferable.

As the silicone adhesive base, compounds having a main ingredient of a polyorganosiloxane or a polydimethylsiloxane are used.

Two or more of the above-mentioned adhesive bases may be mixed for use, and a content of the adhesive base may be usually 5-90% by mass, 10-70% by mass, and furthermore 10-50% by mass relative to the total amount, in consideration of formation of the drug layer and sufficient skin permeability rate of donepezil.

The drug layer may further contain a plasticizer. The plasticizer is exemplified by petroleum-based oils such as paraffinic process oil, naphthenic process oil and aromatic process oil; squalane; squalene; vegetable oils such as olive oil, camellia oil, castor oil, tall oil and peanut oil; silicone oil; dibasic acid esters such as dibutylphthalate and dioctylphthalate; liquid rubbers such as polybutene and liquid isoprene rubber; liquid fatty acid esters such as isopropyl myristate, hexyl laurate, diethyl sebacate and diisopropyl sebacate; diethylene glycol; polyethyleneglycol; glycol salicylate; propylene glycol; dipropylene glycol; triacetin; triethyl citrate; crotamiton and the like. Among them, liquid paraffin, liquid polybutene, isopropyl myristate, diethyl sebacate and hexyl laurate are preferable, and liquid polybutene, isopropyl myristate and liquid paraffin are particularly preferable. Two or more of these plasticizers may be mixed for use.

A content of the plasticizer may be usually 10-70% by mass, 10-60% by mass, and furthermore 10-50% by mass relative to the total amount in consideration of sufficient skin permeability rate of donepezil and conservation of sufficient shear adhesion as a transdermal preparation.

Furthermore the drug layer may contain a tackifying resin. The tackifying resin includes a rosin, a glycerol ester of the rosin, a hydrogenated rosin, a rosin derivative such as a glycerol ester of the hydrogenated rosin and a pentaerythritol ester of the rosin, an alicyclic saturated hydrocarbon resin such as ARKON P100 (Trade name; Arakawa Chemical Industries, Ltd.), an aliphatic hydrocarbon resin such as QUINTONE B170 (Trade name; ZEON CORPORATION JAPAN), a terpene resin such as Clearon P-125 (Trade name; YASUHARA CHEMICAL CO.,LTD.), a maleic acid resin, etc. Among them, the glycerol ester of the hydrogenated rosin, the alicyclic saturated hydrocarbon resin, the aliphatic hydrocarbon resin and the terpene resin are particularly preferable.

A content of the tackifying resin may be usually 5-70% by mass, 5-60% by mass, furthermore 10-50% by mass relative to the total amount, in consideration of sufficient adhesibility as a transdermal preparation and skin irritation while peeling off the transdermal preparation.

The backing is not particularly limited as long as it is suitable for backinging the drug layer, and stretch or non-stretch backing can be used. Films or sheets of polyethylene, polypropylene, polybutadiene, ethylene-vinyl acetate copolymer, polyvinyl chloride, polyester, nylon, polyurethane and the like, and their laminated bodies, porous materials, foams, fabrics and non-woven fabrics, their laminated products and the like can be used. In the drug layer of the plaster, side opposite to the side contacting the backing may be laminated with a release liner which is peeled off for use before being applied to an affected area. As the release liner, polyethylene, polypropylene, polyester, polyethylene terephthalate, and their materials subjected to release treatment with silicone, and release sheet, etc. can be used.

Subsequently, a manufacturing method of the plaster will be explained. In the case of the plaster using an acrylic adhesive base, a drug, oleic acid if needed, a sterol compound and an adhesive base are dissolved or dispersed in a solvent, and the resulting solution or dispersion liquid is either directly applied on the surface of a backing and dried to form a drug layer with a thickness of 30-200 µm, or the above-mentioned solution or dispersion liquid is firstly applied on a sheet or a film subjected to release treatment and dried to form an adhesive layer and thereafter the obtained adhesive layer is transferred to a backing by compression. Next, the side opposite to the side contacting the backing in the drug layer is covered with a release liner, and by being cut into the appropriate size, a plaster is obtained. It should be noted that the order in which each component are added in the above-mentioned manufacturing method is merely an example, and is not limited to the mentioned order. Solvent to be used in this manufacturing method is not particularly limited as long as it is an organic solvent compatible with all the blending components such as adhesive base and drugs, and as a solvent, for example, aromatic hydrocarbons such as toluene, benzene and xylene; esters such as ethyl acetate; halogenated hydrocarbons such as carbon tetrachloride, chloroform and methylene chloride can be used.

In the case of the plaster using a rubber adhesive base, an adhesive base, a plasticizer if needed and a tackifying agent are mixed while heating by using a blender such as a kneader and a mixer. Subsequently, the drug, oleic acid if needed, and a sterol compound are added and uniformly dispersed, the obtained liquid is either directly spread on a backing, or firstly spread on a sheet or a film subjected to release treatment and then transferred to a backing by compression. Next, the side opposite to the side contacting the backing in the drug layer is covered with a release liner, and by being cut into the appropriate size, a plaster is obtained. It should be noted that the order in which each component are added in the above-mentioned manufacturing method is merely an example, and is not limited to the mentioned order.

The transdermal preparation having the above-mentioned constructions can be manufactured by any of the traditionally known methods. For example, the adhesive base containing donepezil is melted by heating and is applied on a release sheet or a backing, then is stuck with a backing or a release sheet to obtain the preparation. In addition, the preparation can be obtained by dissolving adhesive base component containing donepezil in a solvent such as toluene, hexane or ethyl acetate, and spreading the obtained liquid on a release sheet or a backing, drying to remove the solvents, and laminating with a backing or a release sheet.

As another embodiment, an ointment of the transdermal preparation will be explained below. The ointment contains one or more drugs selected from donepezil and pharmaceutically acceptable salts thereof, and a skin irritation-reducing agent. As the skin irritation-reducing agent, one or more sterol compounds selected from cholesterol, cholesterol derivatives and cholesterol analogs are preferable. Furthermore, a transdermal absorption enhancer such as oleic acid may be further contained.

It is preferable that the ointment is substantially free from moisture. "Substantially free from moisture" means that the ointment is made up of non-aqueous materials. However, it is acceptable that the ointment contains only 1% by mass or less of water (moisture) derived from its raw materials or manufacturing environment.

The ointment contains higher fatty acids such as myristic acid or their esters, waxes such as spermaceti, surfactants such as polyoxyethylene, hydrocarbons such as a hydrophilic Vaseline, in addition to the drug, oleic acid if needed, and a sterol compound. The ointment can be manufactured by adding 5-15% by mass of a higher fatty acid or its ester, 1-10% by mass of a surfactant, 0.1-30% by mass of the drug, 0.1-30% by mass of the sterol compound, 4-10% by mass of waxes and 50-90% by mass of hydrocarbons based on the total amount, heating them until the whole components in the solution become transparent, uniformly mixing them using a homo-mixer, and cooling them to the room temperature while stirring.

To these transdermal preparations, various pharmaceutically acceptable additives, for example stabilizers, antioxidants, perfumes, fillers and transdermal absorption enhancers can be added within the ranges not impairing the object of the present invention.

Moreover, another embodiment of the present invention provides a manufacturing method of the transdermally absorbable donepezil-containing preparation allowing reduction in skin irritation, and a method for reducing skin irritation of a transdermal preparation, which comprise a step of adding one or more drugs selected from donepezil and its pharmaceutically acceptable salts, and a skin irritation-reducing agent to the transdermal preparation.

### Examples

Hereinafter, the present invention will be more specifically explained by showing examples, but is not limited to these examples, and various modifications are possible without departing from the technical ideas of the invention.

### (Manufacture Examples)

Transdermally absorbable donepezil-containing preparations were prepared. Materials were mixed at the quantities as described in Table 1 to provide an application liquid. The application liquid was spread on a polyethylene terephthalate film subjected to release treatment, and solvent was removed the by drying with a hot air to provide a drug layer, on which a backing (Sand matte PET, Teijin Dupont Films Japan Limited) was laminated. Then, after being appropriately cut, transdermally absorbable donepezil-containing preparations in Examples 1-9 and Comparative Example 1 were obtained. It should be noted that the ratios in Table 1 are relative to the total amount (% by mass) of the transdermally absorbable donepezil-containing preparation.

**[Table 1]**

| | | EXAMPLE 1 | EXAMPLE 2 | EXAMPLE 3 | EXAMPLE 4 | EXAMPLE 5 | EXAMPLE 6 | EXAMPLE 7 | EXAMPLE 8 | EXAMPLE 9 | COMPARATIVE EXAMPLE 1 |
|---|---|---|---|---|---|---|---|---|---|---|---|
| Acrylic adhesive base | Duro-Tak 387.2516 | 30 | 25 | - | - | - | - | - | - | - | - |
| | Duro-Tak 387-2287 | - | - | 30 | - | - | - | - | - | - | - |
| SIS | | - | - | - | 15 | 15 | 15 | 15 | 15 | 15 | 15 |
| PIB | | - | - | - | 5 | 5 | 5 | 5 | 5 | 5 | 5 |
| Alicyclic saturated hydrocarbon resin | | 25 | 29.5 | 27 | - | 35.5 | 32 | 35 | 32 | 30 | 37 |
| Hydrogenated rosin ester | | - | - | - | 35 | - | - | - | - | - | - |
| Liquid paraffin | | 27 | 27 | 22 | 27 | 30 | 32 | 30 | 32 | 32 | 30 |
| Oleic acid | | 5 | 5 | 5 | - | 6 | 5 | 6 | 6 | 5 | 5 |
| Isopropyl myristate | | - | 5 | - | 5 | - | - | - | - | - | - |
| Polyoxyethylene laurylether | | - | - | 5 | - | - | - | - | - | - | - |
| Cholesterol | | 5 | 0.5 | 3 | 5 | 0.5 | 3 | 1 | 2 | 5 | - |
| Donepezil hydrochloride | | 8 | 8 | 8 | 8 | 8 | 8 | 8 | 8 | 8 | 8 |

### (Experiment 1) Skin permeability test in a hairless mouse

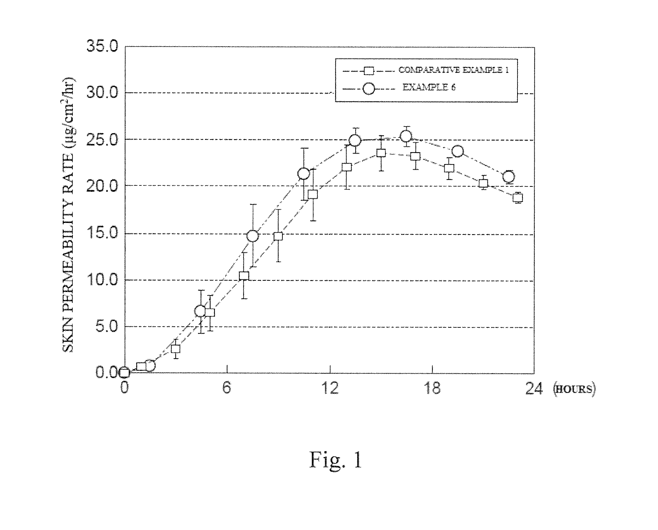

The skin of a hairless mouse was peeled from side of the body, the transdermally absorbable donepezil-containing preparations (about 3 cm²) in Example 6 and Comparative example 1 were applied to the horny layer side, and the skin was set on a flow-through cell, in which warm water (37°C) was circulated around the outer circumference, with the dermis side facing to the receptor side. Through the use of a phosphate buffered saline (PBS, pH 7.4) in the receptor layer, sampling was carried out at a flow rate of about 5 mL/hr every 3 hours for the transdermal preparation in Example 6, or every 2 hours for the transdermal preparation in Comparative example 1, up to 24 hours. A flow volume of the resulting receptor solution was accurately measured, and a drug concentration of donepezil was measured by high-performance liquid chromatography, and then a skin permeability rate of donepezil per hour was calculated. The results are shown in Fig. 1. The transdermally absorbable donepezil-containing preparation in Example 6 exhibited higher skin permeability rate than that in Comparative example 1.

### (Experiment 2) Production decrease in skin irritation mediator by cholesterol in human epidermal cells

There was used a human three-dimensional epidermal culture model in which human normal epidermal cells were multilayer-cultured (LabCyte EPI-MODEL, Japan Tissue Engineering Co., Ltd.). LabCyte is a cultured skin cultured in a transwell. For the experiment, LabCyte was used with the transwell.

A cholesterol (Wako Pure Chemical Industries, Ltd.) was used as a test article. Donepezil hydrochloride was used as the drug. Olive oil (Wako Pure Chemical Industries, Ltd.) was used as a vehicle.

Tests were conducted by the following group composition.
* Drug Alone Group: A group with addition of 4x10⁻⁴ M of donepezil hydrochloride alone dispersed in a vehicle, as a test solution.
* Cholesterol Addition Group: A group with addition of 4x10⁻⁴ M of donepezil hydrochloride and 1% by mass of cholesterol which were dispersed in a vehicle, as a test solution.

The test solutions of each group were added to the cells, the culture after being incubated for 48 hours were collected, and the production amounts of skin irritation mediators were measured. PGE2, IL-1α, IL-6 and IL-8 were measured as the skin irritation mediators. The skin irritation mediators were measured by using a commercially available ELISA kit (R&D Systems, Inc.). The relative production amount of the skin irritation mediator in the Cholesterol Addition Group was calculated as below, when the production amount of the skin irritation mediators in Drug Alone Group was set to 100.

Relative production amount = (production amount of the mediator in Cholesterol Addition Group) / (production amount of the mediator in Drug Alone Group) x 100

The results are shown in Table 2. According to Table 2, cholesterol exhibited effects of decreasing the production amounts of skin irritation mediators by donepezil hydrochloride.

**[Table 2]**

| DRUG | RELATIVE PRODUCTION AMOUNT OF SKIN IRRITATION MEDIATOR WHEN PRODUCTION AMOUNT OF SKIN IRRITATION MEDIATOR IN THE GROUP WITH ADDITION OF DRUG ALONE IS SET TO 100 | | | |
|---|---|---|---|---|
| | PGE2 | IL-1α | IL-6 | IL-8 |
| Donepezil hydrochloride | 33 | 0 | 85 | 59 |

### (Experiment 3) Primary skin irritation test in a rabbit

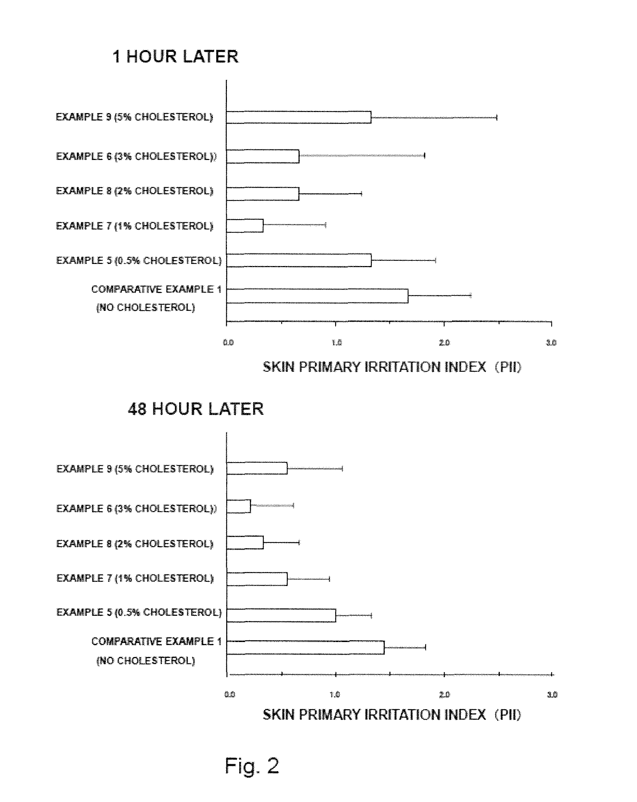

The transdermally absorbable donepezil-containing preparations having a size of 2 cm x 2 cm in Examples 5-9 and Comparative example 1 were attached to a shaved dorsal skin of rabbit. The preparation was peeled 24 hours after the attachment, the formation of skin erythema, crust and swelling were macroscopically observed 1 hour and 48 hours after peeling, and were scored based on the evaluation standard of Draize et al. shown in Table 3 and then the skin primary irritation index (PII) were calculated. The results are shown in Fig. 2. The PII of transdermally absorbable donepezil-containing preparation with the addition of cholesterol was decreased, and when the content of cholesterol ranges 0.5-5% by mass, the PII of the preparation was sufficiently decreased in comparison with Comparative example 1 without cholesterol.

**[Table 3]**

| SKIN ERYTHEMA AND CRUST FORMATION | SCORE |
|---|---|
| No skin erythema | 0 |
| Slight skin erythema (barely visible) | 1 |
| Clear skin erythema | 2 |
| Moderate to severe skin erythema | 3 |
| Severe skin erythema (beet-like red) to slight crust formation (deeply invasive) | 4 |
| | |

| SWELLING FORMATION | SCORE |
|---|---|
| No swelling | 0 |
| Slight swelling (barely visible) | 1 |
| Mild swelling (clearly visible margin by distinct prominence) | 2 |
| Moderate swelling (about 1 mm of prominence) | 3 |
| Severe swelling (about 1 mm of prominence and spread over a range of exposure) | 4 |

### Industrial Applicability

According to an embodiment of the present invention, there can be provided the transdermal preparation comprising one or more drugs selected from donepezil and pharmaceutically acceptable salts thereof, which has low skin irritation as well as a sufficient skin permeability rate of donepezil. According to another embodiment of the present invention, there can be provided a manufacturing method of a transdermally absorbable donepezil-containing preparation with reduced skin irritation and a method for reducing skin irritation of a transdermal preparation, which comprise a step of adding one or more drugs selected from donepezil and pharmaceutically acceptable salts thereof, and a skin irritation-reducing agent to the transdermal preparation.

## Claims

1. A transdermal preparation comprising one or more drugs selected from donepezil and pharmaceutically acceptable salts thereof, and a skin irritation-reducing agent comprising one or more sterol compounds selected from cholesterol, cholesterol derivatives and cholesterol analogs,
wherein the cholesterol derivative is an acylcholesterol which is an ester compound in which a fatty acid binds to the part of the hydroxy group and wherein the cholesterol analog is selected from the group consisting of sitosterol, stigmasterol, fucosterol, spinasterol, campesterol, brassicasterol and ergosterol.

2. The transdermal preparation according to claim 1, wherein content of the skin irritation-reducing agent is 0.5-5% by mass relative to total amount of the transdermal preparation.

## Patentansprüche

1. Eine transdermale Zubereitung, umfassend ein oder mehrere Arzneimittel ausgewählt aus Donepezil und pharmazeutisch verträglichen Salzen davon und ein Mittel, das Hautreizungen reduziert, umfassend eine oder mehrere Sterin-Verbindungen ausgewählt aus Cholesterin, Cholesterin-Derivaten und Cholesterin-Analoga,
wobei das Cholesterin-Derivat ein Acylcholesterin ist, welches eine Esterverbindung, in welcher eine Fettsäure an den Teil der Hydroxygruppe bindet, ist, und wobei das Cholesterin-Analogon ausgewählt ist aus der Gruppe bestehend aus Sitosterin, Stigmasterin, Fucosterin, Spinasterin, Campesterin, Brassicasterin und Ergosterin.

2. Die transdermale Zubereitung gemäß Anspruch 1, wobei der Gehalt des Mittels, welches die Hautreizung reduziert, 0,5 bis 5 Massen-% im Verhältnis zur Gesamtmenge der transdermalen Zubereitung beträgt.

## Revendications

1. Préparation transdermique comprenant un ou plusieurs médicaments choisis parmi le donépézil et ses sels pharmaceutiquement acceptables, et un agent réduisant l'irritation cutanée comprenant un ou plusieurs composés de stérol choisis parmi le cholestérol, les dérivés du cholestérol et les analogues du cholestérol,
le dérivé du cholestérol étant un acylcholestérol qui est un composé d'ester dans lequel un acide gras se lie à la partie du groupe hydroxy, et l'analogue du cholestérol étant choisi dans le groupe constitué par le sitostérol, le stigmastérol, le fucostérol, le spinastérol, le campestérol, le brassicastérol et l'ergostérol.

2. Préparation transdermique selon la revendication 1, dans laquelle la teneur de l'agent réduisant l'irritation cutanée est de 0,5 à 5 % en masse par rapport à la quantité totale de la préparation transdermique.
